# FASCICULE DE BREVET EUROPEEN

(11) **EP 3 071 246 B1**
(45) Date de publication et mention de la délivrance du brevet: **02.01.2019**
(21) Numéro de dépôt: 14824505.3
(22) Date de dépôt: 21.11.2014
(51) Int. Cl.: A61L 27/16, A61L 27/18, A61F 2/08, C08L 25/04, C08L 67/04

(54) **LIGAMENT PROTHÉTIQUE BIOMIMÉTIQUE RÉSORBABLE**
RESORBIERBARE BIOMIMETISCHE LIGAMENTPROTHESE
RESORBABLE BIOMIMETIC PROSTHETIC LIGAMENT

(30) Priorité: 22.11.2013 FR 1361522
(43) Date de publication de la demande: 28.09.2016
(73) Titulaire: L.A.R.S. - laboratoire d'application et de Recherche Scientifique, 21560 Arc-sur-Tille (FR)
(72) Inventeur: BRULEZ, Bernard, 52400 Bourbonne Les Bains (FR); MIGONNEY, Véronique, 95600 Eaubonne (FR); GUILARD, Roger, 21121 Fontaine Les Dijon (FR)
(74) Mandataire: Oudin, Stéphane
(86) Numéro de dépôt international: PCT/FR2014/052992
(87) Numéro de publication internationale: WO 2015/075397

(56) Documents cités:
- FR-A1- 2 850 026

## Description

### Domaine technique

La présente invention concerne un ligament prothétique biomimétique résorbable ainsi que son procédé d'obtention.

### Technique antérieure

La rupture du ligament croisé antérieur (LCA) du genou est une affection fréquente avec une incidence évaluée à 1 pour 3 000 habitants par an aux Etats-Unis et en Europe. Elle survient essentiellement chez des sujets sportifs : ainsi, l'histoire de la rupture du LCA débute-telle dans plus de 65% des cas par un accident sportif (ski, football, rugby, sports de combat). L'âge moyen au moment de l'accident est compris entre 20 et 29 ans, et 70% des patients atteints ont entre 20 et 40 ans.

Le LCA est un élément essentiel de stabilisation du genou. Une fois rompu, en raison de sa localisation intra-articulaire et de la pauvreté de sa vascularisation, il ne cicatrise pas spontanément et l'évolution se fait vers une rétraction et une dégénérescence des extrémités rompues. La laxité du genou qui en résulte entraine un retentissement fonctionnel avec une instabilité qui va gêner ou empêcher la pratique des activités sportives voire de la vie courante. Par ailleurs, cette laxité favorise au long cours la survenue de lésions méniscales puis une dégradation arthrosique du genou qui est observée à 30 ans chez 85 % des sujets atteints (rapport de la HAS, juin 2008). Pour toutes ces raisons, le recours à une intervention chirurgicale est fréquent, en particulier chez les sujets jeunes. En France, 35 732 patients ont subi en 2010, une intervention chirurgicale à la suite d'une rupture du LCA (Source PMSI 2010).

Les techniques chirurgicales usuelles reposent sur le remplacement du LCA par un transplant autologue : le tendon rotulien (technique de Kenneth Jones) ou les tendons des muscles droits internes et demi-tendineux (technique du DIDT). L'utilisation d'une autogreffe n'est cependant pas dénuée d'inconvénients (morbidité liée au prélèvement tissulaire, défaut d'ancrage du greffon au niveau osseux et taux de rupture encore trop important avec un taux d'échec estimé à environ 15%) et induit des délais de récupération importants pour la reprise des activités sportives (> 6 mois).

L'alternative consiste à remplacer le ligament rompu par une prothèse ligamentaire synthétique qui diminue la iatrogénicité du geste chirurgical et offre un soutien mécanique immédiat. Cette dernière solution est proposée par certains chirurgiens aux sportifs de haut niveau pour lesquels la reprise fonctionnelle doit être rapide et aux patients atteints de lésions multi-ligamentaires moins fréquentes mais plus graves. Dans ce dernier cas, l'absence de transplant en nombre suffisant peut conduire à l'utilisation d'allogreffes présentant un risque de transmission virale ou à utiliser, pour le ligament croisé postérieur, une prothèse ligamentaire synthétique comme tuteur de cicatrisation. Il existe également un intérêt économique à cette utilisation par la diminution des coûts indirects liés aux périodes de réadaptation fonctionnelle et d'arrêt d'activité.

Le développement des ligaments artificiels dans les années 1970-80 avait pour ambition de pallier les insuffisances et complications liées aux autogreffes et allogreffes ligamentaires. De nombreux ligaments artificiels ont été proposés, d'abord en carbone, puis en poly(éthylène), en poly(éthylène téréphtalate) (Leeds-Keio ligament), en poly(propylène) (Kennedy Ligament Augmentation Device), ou en poly(tetrafluoroéthylène) (Gore-tex, ou ABC Surgicraft). Si les résultats de ces ligaments dans le traitement des ruptures du LCA étaient bons à court terme, le choix des matériaux était totalement inadéquat et de fait leur faible résistance à l'abrasion, leur taux élevé de rupture en fatigue et leur faible intégration ont naturellement favorisé les échecs constatés à moyen terme. La production de débris ligamentaires par fragmentation fut la source de synovites inflammatoires et de lésions chondrales définitives. L'examen histologique des ligaments explantés a en outre montré que la colonisation tissulaire était inhomogène et qu'elle constituait plus un élément de déstructuration qu'un élément de renfort mécanique. Après une phase d'enthousiasme initial, l'usage de ces ligaments s'est donc progressivement limité à une utilisation comme matériau de renforcement d'une structure ligamentaire autologue plutôt que comme véritable substitut. Les mauvais résultats de ces premiers ligaments ont entrainé leur non-recommandation dans le cadre des ligamentoplasties de première intention du croisé antérieur en France.

Au cours des années 1990 à 2000, une 2ème génération de ligament synthétique a vu le jour et a permis de proposer une solution innovante dans ce domaine spécifique (Ligaments LARS). La prothèse LARS (ligament artificiel en PET) qui constitue par sa structure innovante la seconde génération des prothèses ligamentaires est l'un des ligaments artificiels les plus utilisés depuis une quinzaine d'années. Toutefois, dans la mesure où personne ne connaît aujourd'hui l'impact à long terme de la présence d'une structure synthétique dans l'articulation du genou, des ligaments artificiels de troisième génération ont également été proposés. Ces ligaments artificiels bioactifs utilisables sans réaction hostile de l'hôte, décrit dans le brevet FR0300495, permettent d'améliorer la colonisation tissulaire et fonctionnalité des tissus et l'ancrage osseux.

Afin de pallier ces insuffisances, la présente demande se propose de mettre au point un ligament bioactif et biodégradable (bio-hybride) qui ne présente aucun des défauts des ligaments actuels et qui constitue un dispositif médical facilement manipulable par le praticien et induisant une véritable régénération du tissu lésé.

### Résumé de l'invention

Ainsi la présente invention concerne notamment une prothèse de ligament artificiel remarquable en ce qu'elle comprend une nappe constituée en tout ou partie de fibres biodégradables et résorbables et avantageusement de PCL comme décrites dans les revendications.

Selon un mode de réalisation préféré de l'invention, ledit ligament artificiel est un ligament articulaire ou péri-articulaire. Selon un mode de réalisation tout à fait préféré, ledit ligament artificiel est un ligament croisé antérieur ou postérieur.

Avantageusement, ladite prothèse de ligament est constituée de ladite nappe roulée ou pliée sur elle-même, laquelle nappe comporte tout à fait avantageusement deux parties extrêmes intra-osseuses et une partie intermédiaire intra-articulaire. Ladite partie intermédiaire est préférentiellement constituée par un écheveau de fils de trame longitudinaux, adjacents et non liés entre eux transversalement. Au montage du ligament, on donne une torsion longitudinale à chaque fil actif, aboutissant à un ligament dextrogyre ou lévogyre reproduisant le vrillage naturel des ligaments en flexion.

Dans le cadre de la présente invention, le terme « biodégradable » fait référence aux matériaux capables de se dégrader une fois en place dans l'organisme. Selon un mode de réalisation préféré de l'invention, le terme « biodégradable » fait référence aux fibres capables de perdre entre 1 et 100% de leurs constituants dans une période d'exposition aux conditions physiologiques comprises entre 1 mois et 4 ans. Selon un mode de réalisation tout à fait préféré de l'invention le terme biodégradable fait référence aux matériaux choisis dans le groupe comprenant la poly ε-caprolactone (PCL), les copolymères de ε-caprolactone et d'acide lactique (L et D)ou d'acide glycolique, les copolymères des acides lactiques et glycoliques (L et D), les polydioxanone, les polyhydroxyalcanoate et les copolymères de ces différentes molécules.

Tout à fait avantageusement, lesdites fibres biodégradables sont des fibres de PCL. Ce type de ligament en PCL, matériau dont la biocompatibilité est bien connue, présente une résistance extrêmement élevée aux efforts de traction, flexion et torsion.

La polycaprolactone (PCL) a largement été utilisée dans les années 70-80 dans le domaine des fils de sutures biodégradables, et son utilisation a peu à peu diminué au profit de polyesters plus rapidement résorbables, comme le PLGA. La PCL est un polymère semi-cristallin à haut degré de cristallinité (∼50%) qui possède une température de transition vitreuse Tg de -60°C et une température de fusion Tf de 60°C. Ainsi, lors de son usage à 37°C, les chaînes macromoléculaires de PCL se trouvent dans un état hautement « flexible » permettant son utilisation pour l'ingénierie tissulaire des tissus mous. En outre, son utilisation dans le domaine biomédical est déjà validée puisqu'un grand nombre de dispositifs de délivrance médicamenteuse ont reçu l'approbation de la FDA et le marquage CE.

La PCL se dégrade lentement (jusqu'à 4 ans suivant la masse molaire et la morphologie du matériau) et ne génère pas d'environnement acide extrême durant sa dégradation contrairement au PLGA. Ainsi, la PCL subit une dégradation en deux étapes : tout d'abord, la dégradation hydrolytique jusqu'à une diminution de la masse molaire à 3000 g.mol-1 ; puis la dégradation intracellulaire qui intervient après phagocytose des petits fragments de PCL. La prothèse de ligament selon l'invention est un ligament artificiel biodégradable et "biointégrable" permettant de lever toutes les appréhensions et les incertitudes dues aux supports synthétiques non dégradables. C'est une structure prothétique inspirée et proche du tissu natif, biodégradable tout en étant stérilisable. Elle peut être ensemencée ou non pour faciliter la formation de tissus fonctionnels par une activité cellulaire et tissulaire contrôlées, et présentant les propriétés mécaniques requises.

La prothèse selon l'invention est lentement résorbable afin d'être progressivement remplacée par un tissu fonctionnel identique à celui du ligament natif.

Selon un mode de réalisation préféré de l'invention, ladite fibre biodégradable à un diamètre compris entre 1 et 400µm.

Selon un mode de réalisation préféré de l'invention ladite fibre a une masse molaire comprise entre 1 et 200000 g/mol.

Selon un mode de réalisation préféré de l'invention, ladite nappe est constituée totalement de fibres de PCL.

Lesdites fibres biodégradables sont rendues biologiquement actives par greffage d'un polymère. Ainsi, lesdites fibres biodégradables comprennent des polymères biologiquement actifs.

Ledit polymère biologiquement actif est du poly(styrène sulfonate de sodium). Ainsi, la prothèse ligamentaire selon l'invention est propice à l'adhésion, à la prolifération, à la colonisation, à la différenciation cellulaire ainsi qu'à la production d'une matrice extracellulaire pour re-créer un tissu fonctionnel.

Afin d'assurer une bonne « réhabitation » fibroblastique des prothèses selon l'invention, la présente invention propose un procédé de fonctionnalisation biomimétique desdites prothèses, procédé leur conférant notamment la capacité de mimer les matériaux vivants afin d'améliorer leur intégration biologique.

Ainsi, la présente invention concerne également un procédé de traitement de prothèses artificielles en fibres biodégradables, afin de leur conférer la capacité de mimer les matériaux vivants, ledit procédé de fonctionnalisation biomimétique étant remarquable en ce qu'il comporte au moins une étape de greffage de polymères ou copolymères biologiquement actifs à la surface de la fibre desdites prothèses, laquelle étape de greffage consiste à réaliser une peroxydation de la surface par ozonation suivie d'une polymérisation radicalaire d'une solution d'au moins un monomère.

Selon un mode de réalisation préféré de l'invention, la durée d'ozonation pour une teneur d'ozone de l'ordre de 50g/cm3 est comprise entre 5 et 90 min.

Selon un autre mode de réalisation préféré de l'invention, le monomère est du styrène sulfonate de sodium.

Selon encore un mode de réalisation préféré de l'invention, la solution de monomères a une concentration en monomère(s) comprise entre 5% et k%, où k est une concentration proche de la solubilité limite du ou des monomère(s)dans la solution.

Selon un autre mode de réalisation préféré de l'invention, l'étape de greffage est précédée par une étape supplémentaire de préparation de la surface de la fibre en milieu solvant apte à modifier la surface par gonflement seulement, ou en milieu solvant puis en milieu aqueux.

Selon un autre mode de réalisation préféré de l'invention, le milieu solvant est constitué d'éther éthylique, de DMSO, d'hexane et/ou d'éther éthylique.

Dans le cas de fibres en PCL, le milieu solvant est avantageusement constitué d'éther éthylique.

Selon un autre mode de réalisation préféré de l'invention, le milieu solvant est constitué par au moins un solvant apte à modifier la surface par gonflement.

Selon un autre mode de réalisation préféré de l'invention, le solvant apte à modifier la surface par gonflement est du type éther cyclique ou aliphatique ayant une toxicité faible ou nulle.

Selon un autre mode de réalisation préféré de l'invention, le solvant apte à modifier la surface par gonflement est choisi dans le groupe de solvants suivants : tétrahydrofuranne (THF), diméthylsulfoxyde (DMSO), N,N-diméthylformamide (DMF), N,N-diméthylacétamide (DMA), N-méthylpyrrolidone (NMP).

Selon un autre mode de réalisation préféré de l'invention, l'étape de préparation de la surface en milieu aqueux consiste à traiter la surface en polyester à chaud avec une solution aqueuse de sels de carbonates alcalins ou alcalino-terreux, comme par exemple Na₂CO₃ ou CaCO₃, afin d'éliminer les résidus de fabrication du polyester présents à sa surface.

Selon un autre mode de réalisation préféré de l'invention ledit procédé comporte une étape supplémentaire d'imprégnation de la prothèse après l'étape de greffage par un ou plusieurs agents biochimiques favorisant la colonisation par des fibroblastes.

Selon enfin un autre mode de réalisation préféré de l'invention, l'agent biochimique est une protéine de la famille des fibronectines et/ou du collagène de type I et/ou III.

Le procédé selon l'invention est ici appliqué à des prothèses de ligament déjà formées ou encore aux nappes comprenant des fibres biodégradables entrant dans la fabrication desdites prothèses.

D'autres avantages et caractéristiques ressortiront mieux de la description qui va suivre, de la variante d'exécution complète, donnée à titre d'exemple non limitatif, du ligament et du procédé selon l'invention.

### Description des modes de réalisation

### Elaboration des nappes biodégradables en PCL

Les fibres de PCL peuvent préférentiellement être obtenues par extrusion et notamment par extrusion-soufflage, extrusion-formage et/ou extrusion-filage.

Les fibres biodégradables de PCL peuvent par ailleurs être obtenues par la technique dite « d'electrospinning » permettant l'obtention de fibres à partir d'une solution de polymère et enfin des nappes de polycaprolactone (PCL). Le principe de cette technique réside dans l'application d'une haute tension sur une solution de polymère engendrant alors la formation d'un jet qui, une fois déposé sur un collecteur, forme un tapis fibreux. La versatilité de cette technique permet de fabriquer des structures fibreuses dont le diamètre des fibres est ajusté en influant sur la concentration, la composition et le débit de la solution.

### Etape 1 : Préparation de la surface en PCL

### 1A) En milieu solvant :

Cette étape dite de désensimage est nécessaire afin d'éliminer les graisses et impuretés incorporées lors de la fabrication de la trame en PCL servant de structure au ligament. Elle permet ainsi d'éviter des réactions pathologiques de type synovite aiguë lors de l'implantation chez le patient. En outre, cette étape permet d'assurer une croissance des fibroblastes sur cette surface de PCL ainsi nettoyée, croissance non observée sur des surfaces non nettoyées.

On distingue trois variantes de cette préparation en milieu solvant, selon la nature du solvant et/ou du surfactant choisi.
- Variante 1 : Désensimage avec un solvant apte à gonfler la surface en PCL :
   L'utilisation d'un solvant apte à gonfler la surface du PCL offre l'avantage d'améliorer le greffage en augmentant le nombre de peroxydes sur la surface traitée lors de l'étape d'ozonation.

En outre, on le choisira dans le groupe de solvant suivant : tétrahydrofuranne (THF), le chloroforme et le dichlorométhane. Ces solvants ont l'avantage de présenter une toxicité faible ou nulle et permettent ainsi une utilisation facile en milieu industriel.

Le traitement se fait par immersion de la PCL dans le solvant pendant une durée de l'ordre de 5 minutes à une heure environ, de préférence en 10 à 25 minutes. Ainsi, à titre d'exemple, on choisira une durée de 15 minutes à température ambiante pour une immersion dans du tétrahydrofuranne (THF).
- Variante 2 : Désensimage avec un solvant et un surfactant. Ce désensimage est préférablement réalisé en présence d'hexane à une température inférieure à 60°C.
- Variante 3 : Désensimage sans gonflement de la surface :
   On applique un minimum de 12 cycles d'extraction dans un appareil de SOHXLET et un contrôle des résidus de corps gras après le 12ème cycle de nettoyage à l'hexane. On fait suivre ces cycles de nettoyage à l'hexane par des cycles de lavage à l'éther éthylique (RPE), trois lavages minimum avec contrôle des résidus après le troisième lavage.

### 1B) En milieu aqueux :

Le but de cette étape optionnelle de préparation de la surface en milieu aqueux est d'éliminer les résidus de fabrication du PCL présent à sa surface. On obtient ainsi une surface parfaitement apprêtée avant ozonation.

Le traitement consiste à laver la PCL dans une solution de carbonate de sodium (Na₂CO₃) à 5% en poids dans l'eau distillée. Ce lavage est effectué à chaud, c'est-à-dire à plus de 60°C et moins de 120°C et de préférence à légère ébullition, soit 100°C ± 5°C, pendant une dizaine de minutes. Bien entendu, tout autre carbonate alcalin ou alcalino-terreux tel que K₂CO₃ ou CaCO₃ peut être utilisé. Le lavage est suivi de rinçages successifs à l'eau distillée jusqu'à ce que le pH de l'eau de rinçage soit revenu à 7.

### 1C) Nettoyage :

Quelles que soient les étapes précédemment effectuées (variante 1 ou 2 de l'étape 1A, puis l'étape 1B, ou seulement l'étape 1A), le produit en PCL est ensuite nettoyé, par exemple par un rinçage à l'éthanol absolu ou au tétrahydrofuranne (THF) suivi d'un séchage en étuve d'une durée de 30 minutes, par exemple.

### Etape 2 : Greffage de polymères ou de copolymères biologiquement actifs sur la surface en PCL :

### 2A) Choix et préparation des monomères :

Les monomères utilisés selon l'invention sont des monomères susceptibles de polymérisation et de copolymérisation radicalaire donnant naissance à des polymères bio-compatibles stimulant la prolifération et la différenciation cellulaire et plus particulièrement celle des fibroblastes. De tels monomères contenant des groupes hydroxyle,carboxylate, phosphonate, sulfonate et sulfate sont, par exemple, décrits dans le brevet US 6.365.692 et peuvent être utilisés selon l'invention seuls ou en mélange. On pourra, par exemple, utiliser de l'acide méthacrylique et du styrène sulfonate, ainsi que leurs mélanges. Avant de les utiliser pour la polymérisation, ces monomères seront purifiés au préalable. Ainsi par exemple, pour du styrène sulfonate de sodium, on le purifie par recristallisation dans un mélange d'eau bidistillée/alcool (10/90,v:v), puis on le dissout à 70°C dans cette solution. On le filtre ensuite sous vide avec un disque en verre fritté d'indice de porosité 3 et on le conserve à 4°C. Les cristaux de sulfonate de sodium formés sont récupérés par filtration et le solide obtenu est séché sous vide à 50°C jusqu'à l'obtention d'un poids constant.

### 2B) Ozonation :

Les prothèses ou les trames en PCL constitutives de ces prothèses préalablement traitées selon l'étape 1 sont introduites dans un dispositif d'ozonation tel que classiquement utilisé.

Par exemple, on pourra utiliser un réacteur tubulaire de 500 cm³ contenant 100 cm³ d'eau bidistillée, lequel réacteur est muni d'un tube plongeur d'alimentation en ozone. On pourra, par exemple, utiliser un flux gazeux équivalent en ozone à 50 g/m³ d'oxygène. Pour une telle quantité d'ozone, la durée optimale d'ozonation du PCL est de 5 à 90 minutes. Les mesures du taux de peroxyde montrent que le taux optimal est obtenu entre 10 et 30 minutes d'ozonation, toujours pour ce même flux d'ozone. On notera également qu'une durée d'ozonation supérieure à 90 minutes dégrade fortement la surface en PCL.

On relèvera par ailleurs l'apport de la variante 2 de l'étape 1A. En effet, l'utilisation d'un solvant apte à gonfler la surface augmente le taux de peroxyde d'un facteur 5, par rapport à l'utilisation d'un solvant sans gonflement. Une fois l'ozonation terminée, les prothèses ou les trames en PCL introduites dans le dispositif d'ozonation sont rincées et nettoyées, par exemple selon le protocole suivant : rincer trois fois à l'eau bidistillée, puis trois fois à l'alcool absolu. Ensuite, sécher à l'étuve à vide pendant 30 minutes à 25°C.

### 2C) Polymérisation :

Le ou les monomères choisis et préparés selon l'étape 2A sont mis en solution dans de l'eau, de préférence bidistillée. On peut choisir toute concentration compatible avec la mise en oeuvre de la réaction de polymérisation radicalaire avec un minimum de 2% en poids. On choisira avantageusement des concentrations proches de la solubilité limite du ou des monomère(s) dans la solution, avec un milieu visqueux favorisant ainsi des réactions de propagation polymérique radicalaire par rapport à des réactions de terminaison. Cela revient à choisir une concentration pondérale k = s - ε, ou s est la solubilité limite et ε vaut 1 à 7% en poids. Ainsi par exemple, dans le cas du polystyrène sulfonate dont la limite de solubilité est de 20% en poids, on choisira une concentration de 15%.

La durée de l'étape de polymérisation dépend de la nature du monomère. Elle est estimée au temps nécessaire à la gélification du milieu à la température de réaction. Ainsi, par exemple, on retiendra pour le polystyrène sulfonate qu'à 50°C, la polymérisation durera 1 heure et qu'à 30°C, elle durera 15 heures.

La réaction de polymérisation est conduite dans une enceinte hermétiquement fermée et débarrassée de tout oxygène, par exemple, en réalisant un bullage à l'argon. On introduit dans cette enceinte la solution de monomères ou de comonomères que l'on veut faire réagir et les prothèses ou bandes de tissus en PCL préalablement ozonées. Le récipient hermétiquement fermé est chauffé au bain-marie à la température et à la durée déterminée comme exposé précédemment.

En fin de réaction, les éléments en PCL qui ont été greffés sont extraits du réacteur. On peut ensuite laver ces matériaux greffés afin d'éliminer notamment des résidus de monomère(s) qui n'ont pas réagi. Par exemple, on peut laver la surface fonctionnalisée plusieurs fois avec un solvant adéquat du ou des monomère(s), de l'eau bidistillée par exemple, et on peut optionnellement finir le lavage avec tout solvant adéquat, de l'éthanol absolu par exemple, afin d'éliminer d'éventuelles traces de monomères et polymères non greffés.

### Etape 3 : Imprégnation avec des agents biochimiques :

Cette étape est optionnelle. Elle vise à renforcer la capacité d'intégration biologique du ligament auquel on a précédemment greffé des polymères biomimétiques tel qu'exposé aux étapes 1 et 2. Ainsi, l'imprégnation de la prothèse par un ou plusieurs agents biochimiques vise à augmenter ces propriétés d'adhérence et de prolifération cellulaire. Ces agents biochimiques favorisant la colonisation par des fibroblastes sont une protéine de la famille des fibronectines et/ou du collagène de type I et/ou III. On utilisera avantageusement un mélange des précédentes protéines, c'est-à-dire un mélange de fibronectines et collagène de type I et/ou III : on observe un effet synergique sur l'adhérence des fibroblastes. L'imprégnation de la prothèse par ces agents pourra, par exemple, être effectuée par trempage dans un bain contenant du collagène.

Il va de soi que cette étape d'imprégnation ne suit pas forcément l'étape de greffage et qu'elle peut s'insérer et s'intercaler entre d'autres étapes de préparation du ligament en fonction de son stade de fabrication. En outre, on fera avantageusement suivre cette étape d'imprégnation par une étape de stérilisation du ligament.

### Stérilisation des nappes et ligaments de PCL

Le choix de la méthode de stérilisation est crucial lors du développement de biomatériaux à base de polyesters hydrolysables et une attention particulière doit y être portée. Les polyesters aliphatiques étant sensibles à l'humidité et la chaleur, les méthodes de stérilisation par autoclave ou chaleur sèche ne sont pas envisageables. En outre, dans ce mode de stérilisation, s'ajoutent des problèmes de toxicité liée à la difficulté d'éliminer totalement les résidus d'oxyde d'éthylène de l'échafaudage biodégradable.

Trois méthodes de stérilisation sont préférentiellement utilisables : la stérilisation à l'éthanol, les rayonnements UV et les radiations beta.

## Revendications

1. Prothèse de ligament artificiel **caractérisée en ce qu'**elle comprend une nappe constituée en tout ou partie de fibres biodégradables et résorbables comprenant un matériau choisi dans le groupe comprenant la PCL, les copolymères de PCL et d'acide lactique (L et D) ou d'acide glycolique, les copolymères des acides lactiques et glycoliques (L et D), les polydioxanone, les polyhydroxyalcanoate et les copolymères de ces différentes molécules, et des polymères biologiquement actifs de styrène sulfonate de sodium.

2. Prothèse de ligament artificiel selon la revendication précédente **caractérisée en ce que** ladite fibre biodégradable et résorbable comprend de la PCL.

3. Prothèse de ligament artificiel selon la revendication précédente **caractérisée en ce que** ladite nappe est constituée totalement de fibres de PCL.

4. Prothèses de ligament artificiel selon l'une des revendications précédentes **caractérisée en ce que** ledit ligament artificiel est un ligament articulaire ou péri-articulaire.

5. Prothèses de ligament artificiel selon l'une des revendications précédentes **caractérisée en ce que** ledit ligament artificiel est un ligament croisé antérieur ou postérieur.

6. Procédé de traitement de prothèses artificielles en fibres biodégradables selon l'une des revendications 1 à 5, afin de leur conférer la capacité de mimer les matériaux vivants, dit procédé de fonctionnalisation biomimétique **caractérisé en ce qu'**il comporte au moins une étape de greffage de polymères ou copolymères biologiquement actifs à la surface desdites prothèses, laquelle étape de greffage consiste à réaliser une peroxydation de la surface par ozonation suivie d'une polymérisation radicalaire d'une solution d'au moins un monomère.

7. Procédé selon la revendication 6 **caractérisé en ce que** la durée d'ozonation pour une teneur d'ozone de l'ordre de 50g/m³ est comprise entre 5 et 90 min.

8. Procédé selon l'une quelconque des revendications 6 ou 7 **caractérisé en ce que** le monomère est du styrène sulfonate de sodium.

9. Procédé selon l'une quelconque des revendications 6 à 7 **caractérisé en ce que** la solution de monomères a une concentration en monomère(s) comprise entre 5% et k%, où k est une concentration proche de la solubilité limite du ou des monomère(s)dans la solution.

10. Procédé selon l'une quelconque des revendications 6 à 8 **caractérisé en ce que** l'étape de greffage est précédée par une étape supplémentaire de préparation de la surface en milieu solvant seulement, ou en milieu solvant puis en milieu aqueux.

11. Procédé selon la revendication 10 **caractérisé en ce que** le milieu solvant est constitué d'hexane ou d'éther éthylique.

12. Procédé selon la revendication 10 **caractérisé en ce que** le milieu solvant est constitué par au moins un solvant apte à modifier la surface par gonflement.

13. Procédé selon la revendication 12 **caractérisé en ce que** le solvant apte à modifier la surface par gonflement est de l'éther.

14. Procédé selon l'une quelconque des revendications 12 ou 13 **caractérisé en ce que** le solvant apte à modifier la surface par gonflement est choisi dans le groupe de solvants suivants : tétrahydrofuranne (THF), diméthylsulfoxyde (DMSO), N,N-diméthylformamide (DMF), N,N-diméthylacétamide (DMA), N-méthylpyrrolidone (NMP).

15. Procédé selon la revendication 10 **caractérisé en ce que** l'étape de préparation de la surface en milieu aqueux consiste à traiter la surface en polyester à chaud avec une solution aqueuse de sels de carbonates alcalins ou alcalino-terreux, comme par exemple Na₂CO₃ ou CaCO₃, afin d'éliminer les résidus de fabrication du polyester présents à sa surface.

16. Procédé selon l'une des revendications 6 à 15 **caractérisé en ce qu'**il comporte une étape supplémentaire d'imprégnation de la prothèse après l'étape de greffage par un ou plusieurs agents biochimiques favorisant la colonisation par des fibroblastes.

17. Procédé selon la revendication 16 **caractérisé en ce que** l'agent biochimique est une protéine de la famille des fibronectines et/ou du collagène de type I et/ou III.

## Patentansprüche

1. Kunstbandprothese, **dadurch gekennzeichnet, dass** dieselbe eine Lage umfasst, die vollständig oder teilweise aus biologisch abbaubaren und resorbierbaren Fasern, umfassend ein Material ausgewählt aus der Gruppe umfassend PCL, Copolymere aus PCL und Milchsäure (L und D) oder Glykolsäure, Copolymere aus Milch- und Glykolsäuren (L und D), Polydioxanon, Polyhydroxyalkanoat und Copolymere aus diesen verschiedenen Molekülen, und aus biologisch aktiven Natriumstyrolsulfonatpolymeren besteht.

2. Kunstbandprothese nach dem vorstehenden Anspruch, **dadurch gekennzeichnet, dass** die biologisch abbaubare und resorbierbare Faser PCL umfasst.

3. Kunstbandprothese nach dem vorstehenden Anspruch, **dadurch gekennzeichnet, dass** die Lage vollständig aus PCL-Fasern besteht.

4. Kunstbandprothesen nach einem der vorstehenden Ansprüche, **dadurch gekennzeichnet, dass** das künstliche Band ein Gelenkband oder periartikuläres Band ist.

5. Kunstbandprothesen nach einem der vorstehenden Ansprüche, **dadurch gekennzeichnet, dass** das Kunstband ein vorderes oder hinteres Kreuzband ist.

6. Verfahren zur Behandlung von Kunstprothesen aus biologisch abbaubaren Fasern nach einem der Ansprüche 1 bis 5, um denselben die Fähigkeit zu verleihen, die lebenden Materialien zu mimen, wobei das Verfahren zur biomimetischen Funktionalisierung **dadurch gekennzeichnet ist, dass** es mindestens einen Schritt des Pfropfens von biologisch aktiven Polymeren oder Copolymeren an die Oberfläche der Prothesen umfasst, welcher Schritt des Pfropfens im Ausführen einer Peroxidation der Oberfläche durch Ozonisation, gefolgt von einer radikalischen Polymerisation einer Lösung mindestens eines Monomers besteht.

7. Verfahren nach Anspruch 6, **dadurch gekennzeichnet, dass** die Ozonisationsdauer für einen Ozongehalt in der Größenordnung von 50 g/m³ im Bereich zwischen 5 und 90 Min. beträgt.

8. Verfahren nach einem der Ansprüche 6 oder 7, **dadurch gekennzeichnet, dass** das Monomer Natriumstyrolsulfonat ist.

9. Verfahren nach einem der Ansprüche 6 bis 7, **dadurch gekennzeichnet, dass** die Monomerlösung eine Konzentration an Monomer(en) im Bereich zwischen 5 % und k % aufweist, wobei k eine Konzentration nahe der Löslichkeitsgrenze des Monomers oder der Monomere in der Lösung ist.

10. Verfahren nach einem der Ansprüche 6 bis 8, **dadurch gekennzeichnet, dass** dem Schritt des Pfropfens ein zusätzlicher Schritt des Vorbereitens der Oberfläche in nur Lösungsmittelmedium, oder in Lösungsmittelmedium und anschließend in wässrigem Medium vorausgeht.

11. Verfahren nach Anspruch 10, **dadurch gekennzeichnet, dass** das Lösungsmittelmedium aus Hexan oder aus Ethylether besteht.

12. Verfahren nach Anspruch 10, **dadurch gekennzeichnet, dass** das Lösungsmittelmedium aus mindestens einem Lösungsmittel besteht, das dazu in der Lage ist, die Oberfläche durch Aufquellen zu modifizieren.

13. Verfahren nach Anspruch 12, **dadurch gekennzeichnet, dass** das Lösungsmittel, das dazu in der Lage ist die Oberfläche durch Aufquellen zu modifizieren, Ether ist.

14. Verfahren nach einem der Ansprüche 12 oder 13, **dadurch gekennzeichnet, dass** das Lösungsmittel, das dazu in der Lage ist, die Oberfläche durch Aufquellen zu modifizieren, aus der Gruppe folgender Lösungsmittel ausgewählt ist: Tetrahydrofuran (THF), Dimethylsulfoxid (DMSO), N,N-Dimethylformamid (DMF), N,N-Dimethylacetamid (DMA), N-Methylpyrrolidon (NMP).

15. Verfahren nach Anspruch 10, **dadurch gekennzeichnet, dass** der Schritt des Vorbereitens der Oberfläche in wässrigem Medium darin besteht, die warme Polyesteroberfläche mit einer wässrigen Alkalicarbonat- oder Erdalkalisalzlösung, wie zum Beispiel Na₂CO₃ oder CaCO₃ zu behandeln, um die Rückstände aus Herstellung des Polyesters, die an dessen Oberfläche vorhanden sind, zu entfernen.

16. Verfahren nach einem der Ansprüche 6 bis 15, **dadurch gekennzeichnet, dass** es einen zusätzlichen Schritt des Imprägnierens der Prothese nach dem Schritt des Pfropfens mit einem oder mehreren biochemischen Mitteln umfasst, die die Besiedelung durch Fibroblasten begünstigen.

17. Verfahren nach Anspruch 16, **dadurch gekennzeichnet, dass** das biochemische Mittel ein Protein aus der Familie der Fibronektine und/oder Kollagen vom Typ I und/oder III ist.

## Claims

1. Artificial ligament prosthesis **characterised in that** it comprises a layer totally or partly consisting of biodegradable and resorbable fibres comprising a material selected from the group comprising PCL, copolymers of PCL and of lactic acid (L and D) or of glycolic acid, copolymers of lactic and glycolic acids (L and D), polydioxanone, polyhydroxyalcanoate and copolymers of these various molecules, and biologically active polymers of styrene sodium sulfonate.

2. Artificial ligament prosthesis as claimed in the preceding claim **characterised in that** said biodegradable and resorbable fibre comprises PCL.

3. Artificial ligament prosthesis as claimed in the preceding claim **characterised in that** said layer is totally constituted of PSL fibres.

4. Artificial ligament prosthesis according to one of the preceding claims **characterised in that** said artificial ligament is an articular or periarticular ligament.

5. Artificial ligament prosthesis according to one of the preceding claims **characterised in that** said artificial ligament is an anterior or posterior cruciate ligament.

6. Method for treating artificial prostheses made of biodegradable fibres according to any of claims 1 to 5, so as to confer on them the capacity to mimic living materials, with said method of biomimetic functionalisation **characterised in that** it comprises at least one step of grafting of biologically active polymers or copolymers to the surface of said prostheses, said step of grafting consists in carrying out a peroxidation of the surface par ozonation followed by a radical polymerisation with a solution of at least one monomer.

7. Method according to claim 6 **characterised in that** the duration of ozonation for an ozone content of about 50g/cm³ is between 5 and 90 min.

8. Method according to any of claims 6 or 7 **characterised in that** the monomer is styrene sodium sulfonate.

9. Method according to any of claims 6 to 7 **characterised in that** the solution of monomers has a concentration in monomer(s) between 5% and k%, where k is a concentration close to the solubility limit of the monomer(s) in the solution.

10. Method according to any of claims 6 to 8 **characterised in that** the step of grafting is preceded by an additional step of preparing the surface in solvent medium only, or in solvent medium then in aqueous medium.

11. Method according to claim 10 **characterised in that** the solvent medium is comprised of hexane or of ethyl ether.

12. Method according to claim 10 **characterised in that** the solvent medium is constituted by at least one solvent able to modify the surface via swelling.

13. Method according to claim 12 **characterised in that** the solvent able to modify the surface via swelling is ether.

14. Method according to any of claims 12 or 13 **characterised in that** the solvent able to modify the surface via swelling is selected from the following group of solvents: tetrahydrofuran (THF), dimethylsulfoxide (DMSO), N,N-dimethylformamide (DMF), N,N-dimethylacetamide (DMA), N-methylpyrrolidone (NMP).

15. Method according to claim 10 **characterised in that** the step of preparing the surface in an aqueous medium consists in hot-treating the polyester surface with an aqueous solution of alkaline or alkaline earth metal carbonate salts, such as for example Na₂CO₃ or CaCO₃, in order to eliminate the manufacturing residue of the polyester present on its surface.

16. Method according to one of claims 6 to 15 **characterised in that** it comprises an additional step of impregnation of the prosthesis after the step of grafting by one or several biochemical agents that favour colonisation by fibroblasts.

17. Method according to claim 16 **characterised in that** the biochemical agent is a protein of the family of fibronectins and/or type I and/or III collagen.
